# EUROPEAN PATENT APPLICATION

(11) **EP 4 681 703 A1**
(43) Date of publication of application: **21.01.2026**
(21) Application number: 24771265.6
(22) Date of filing: 14.03.2024
(51) Int. Cl.: A61K 9/00, A61K 47/06, A61K 47/44, A61K 47/14, A61K 9/06, A61K 31/444, A61P 29/00, A61P 37/00, A61P 17/00, A61P 17/06

(54) **TOPICAL PHARMACEUTICAL FORMULATION COMPRISING KINASE INHIBITOR**

(30) Priority: 15.03.2023 KR 20230034196
(71) Applicant: HK inno.N Corporation, Cheongju-si, Chungcheongbuk-do 28158 (KR)
(72) Inventor: PARK, Mi-Ran, Seoul 04551 (KR); OH, Tackoon, Hwaseong-si Gyeonggi-do 18478 (KR); LEE, Juhyun, Seoul 04551 (KR); LEE, Chung Gi, Seoul 04551 (KR); JEON, Eun Kyung, Hwaseong-si Gyeonggi-do 18446 (KR); JUNG, Jin Woo, Seoul 04551 (KR); HWANG, Do Seok, Seoul 04551 (KR)
(74) Representative: HGF
(86) International application number: PCT/KR2024/095547
(87) International publication number: WO 2024/191266

(57) **Abstract**

The present invention relates to a pharmaceutical preparation for topical use, a use thereof, and a method for preparing the same, in which the pharmaceutical preparation for topical use includes (S)-N-(4-(1-(2-cyanoacetyl)-3-methyl-1,2,3,6-tetrahydropyridin-4-yl)-1H-pyrrolo[2,3-b]pyridin-6-yl)cyclopropanecarboxamide or a pharmaceutically acceptable salt thereof as an active ingredient, and an oleaginous base.

## Description

### Technical Field

The present invention relates to a pharmaceutical preparation for topical use including a protein kinase inhibitor as an active ingredient, a use thereof, and a method for preparing the same.

### Background

Janus kinase (JAK) is an enzyme of an intracellular non-receptor tyrosine kinase family which delivers a cytokine-mediated signal through a JAK-STAT pathway and consists of JAK1, JAK2, JAK3, and TYK2. A JAK inhibitor is involved in hematopoiesis and immunity, and thus has been studied as a therapeutic agent for myeloproliferative tumors, rheumatoid arthritis and other immunity, inflammatory diseases, or the like and has been developed as compounds such as tofacitinib, baricitinib, upadacitinib, etc.

The JAK inhibitor has received attention for its therapeutic effect as much as a biological agent in the treatment of chronic inflammatory diseases, but has caused concerns about long-term safety. Xeljanz (component name: tofacitinib) is the world's first oral JAK inhibitor which effectively inhibits JAK1/3 and has been approved as a therapeutic agent for rheumatoid arthritis (RA), adult active psoriatic arthritis (PsA), adult active ulcerative colitis (UC), etc., but has been found to have a risk for cardiovascular disease, cancer, and thrombus as a result of clinical trials, thus prompting the FDA to warn of the risk. Inhibition of JAK3 has been reported to cause severe combined immune deficiency (SCID) in humans, and JAK2 has been reported to have anemia side effects associated with an erythropoietin (EPO) signal pathway. In order to minimize such problems, the JAK1 inhibitor which selectively inhibits JAK1 has been developed and is expected to reduce side effects in terms of safety.

Meanwhile, in terms of a therapeutic agent for inflammatory skin diseases, the development of topical formulations is also being attempted to further improve safety. Luxolitinib cream and delgocitinib ointment as therapeutic agents for atopic dermatitis have been approved in the United States and Japan, respectively. The topical formulation shows efficacy only at a lesion site rather than in systemic circulation, and thus is expected to greatly ease concerns about side effects described above.

### Detailed Description of the Invention

### Technical Problem

Accordingly, the present invention may provide a pharmaceutical preparation for topical use including a JAK inhibitor, which is a protein kinase inhibitor, as an active ingredient, a use thereof, and a method for preparing the same.

### Technical Solution

A pharmaceutical preparation for topical use according to the present invention may include a therapeutically effective amount of (S)-N-(4-(1-(2-cyanoacetyl)-3-methyl-1,2,3,6-tetrahydropyridin-4-yl)-1H-pyrrolo[2,3-b]pyridin-6-yl)cyclopropanecarboxamide or a pharmaceutically acceptable salt thereof as an active ingredient; and an oleaginous base.

In one example, above (S)-N-(4-(1-(2-cyanoacetyl)-3-methyl-1,2,3,6-tetrahydropyridin-4-yl)-1H-pyrrolo[2,3-b]pyridin-6-yl)cyclopropanecarboxamide, which is an active ingredient included in the pharmaceutical preparation for topical use, may be a compound represented by formula I below.

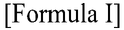

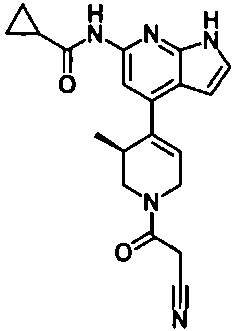

In one example, the pharmaceutical preparation for topical use may include a theoretically effective amount of (S)-N-(4-(1-(2-cyanoacetyl)-3-methyl-1,2,3,6-tetrahydropyridin-4-yl)-1H-pyrrolo[2,3-b]pyridin-6-yl)cyclopropanecarboxamide or the pharmaceutically acceptable salt thereof as an active ingredient, and the oleaginous base.

In one example, the pharmaceutical preparation for topical use may include a therapeutically effective amount of (S)-N-(4-(1-(2-cyanoacetyl)-3-methyl-1,2,3,6-tetrahydropyridin-4-yl)-1H-pyrrolo[2,3-b]pyridin-6-yl)cyclopropanecarboxamide or the pharmaceutically acceptable salt thereof as an active ingredient; the oleaginous base; and at least one pharmaceutically acceptable excipient.

In some embodiments, a formulation of the pharmaceutical preparation according to the present invention may be ointment, cream, lotion, foam or suspension. Particularly, a topical formulation in the present invention may be ointment, cream, foam, and suspension, and more particularly the topical formulation may be the ointment.

In some embodiments, the pharmaceutical preparation may optionally include one or more other pharmaceutically acceptable excipients.

In some embodiments, the oleaginous base may include at least one selected from a semi-solid hydrocarbon mixture, oil, wax, and fatty acid.

In some embodiments, the semi-solid hydrocarbon mixture may include at least one selected from white petrolatum and purified lanolin, but is not limited thereto.

In some embodiments, the oil may include at least one selected from liquid paraffin, squalane, medium chain triglyceride, olive oil, castor oil, soybean oil, and liquid lanolin, but is not limited thereto.

In some embodiments, the wax may include at least one of white wax, paraffin wax, and carnauba wax, but is not limited thereto.

In some embodiments, the fatty acid may include at least one of cocoa butter, shea butter, and lanolin acid, but is not limited thereto.

In one example, the oleaginous base may include white petrolatum, liquid paraffin, white wax, or a combination of two or more thereof.

In one example, the oleaginous base may include white petrolatum and liquid paraffin.

In one example, the oleaginous base may include white petrolatum and white wax.

In one example, the oleaginous base may include white petrolatum, liquid paraffin, and white wax.

In some embodiments, the oleaginous base may be included in an amount of about 45 to about 99.99% by weight with respect to the total 100% by weight of the pharmaceutical preparation.

In some embodiments, a content of the oleaginous base may be about 70 to about 99.95% by weight with respect to the total 100% by weight of the pharmaceutical preparation.

In the present invention, the "pharmaceutically acceptable excipient" may refer to an excipient different from the oleaginous base, and may be defined as not including the oleaginous base. In the present invention, the pharmaceutically acceptable excipient may include skin permeation enhancers, antioxidants, preservatives, chelating agents, emulsifiers, pH adjusters, stabilizers, fragrances, colorants, or a combination of two or more thereof.

In some embodiments, the excipient may include skin permeation enhancers, antioxidants, preservatives, or a combination of two or more thereof.

In some embodiments, the skin permeation enhancer may include a combination of two or more of alcohols, amides, fatty acids, esters, surfactants, essential oils, terpenes, and phospholipids, but is not limited thereto.

In some embodiments, the alcohol may include a saturated fatty alcohol having at least eight carbon atoms. The alcohol may include capric alcohol, lauryl alcohol, myristyl alcohol, cetyl alcohol, stearyl alcohol, or a combination of two or more thereof, but is not limited thereto.

In some embodiments, the amide may include laurocapram, but is not limited thereto.

In some embodiments, the fatty acid, which is a saturated fatty acid or unsaturated fatty acid having seven or more carbon atoms, particularly seven or more and 30 or less carbon atoms, may include caproic acid, caprylic acid, capric acid, lauric acid, myristic acid, palmitic acid, stearic acid, oleic acid, or a combination of two or more thereof, but is not limited thereto.

In some embodiments, the ester may include isopropyl decanoate, isopropyl myristate, isopropyl palmitate, sucrose laurate, or a combination of two or more thereof, but is not limited thereto.

In some embodiments, the surfactant may include sodium lauryl sulfate, vitamin E TPGS, lauryl macrogol glyceride, Span 60, Span 83, or a combination of two or more thereof, but is not limited thereto.

In some embodiments, the essential oil may include eucalyptus oil, ylang ylang oil, Chenopodium oil, or a combination of two or more thereof, but is not limited thereto.

In some embodiments, the terpene may include geraniol, linalool, limonene, menthol, eucalyptol, carvone, squalene, or a combination of two or more thereof, but is not limited thereto.

In some embodiments, the phospholipid may include phosphatidyl choline, but is not limited thereto.

In some embodiments, the skin permeation enhancer may be included in an amount of about 0.1 to about 30% by weight with respect to the total 100% by weight of the pharmaceutical preparation.

In some embodiments, a content of the skin permeation enhancer may be about 1 to about 15% by weight with respect to the total 100% by weight of the pharmaceutical preparation, particularly about 1% by weight, about 2% by weight, about 3% by weight, about 4% by weight, about 5% by weight, about 6% by weight, about 7% by weight, about 8% by weight, about 9% by weight, about 10% by weight, about 11% by weight, about 12% by weight, about 13% by weight, about 14% by weight, or about 15% by weight with respect to the total 100% by weight of the pharmaceutical preparation.

In some embodiments, the preservative may include methyl paraben, propyl paraben, phenoxy ethanol, benzyl alcohol, sodium benzoate, or a combination of two or more thereof, but is not limited thereto.

In some embodiments, the preservative may be included in an amount of about 0.01 to about 5% by weight with respect to the total 100% by weight of the pharmaceutical preparation.

In some embodiments, a content of the preservative may be about 0.05 to about 3% by weight with respect to the total 100% by weight of the pharmaceutical preparation, for example, particularly about 0.05% by weight, about 0.1% by weight, about 0.2% by weight, about 0.3% by weight, about 0.4% by weight, about 0.5% by weight, about 1% by weight, about 1.5% by weight, about 2% by weight, about 2.5% by weight, or about 3% by weight with respect to the total 100% by weight of the pharmaceutical preparation.

In some embodiments, the antioxidant may include tocopherol, tocopherol acetate, BHT, BHA, ascorbyl palmitate, alpha-lipoic acid, silicon dioxide, zinc oxide, or a combination of two or more thereof, but is not limited thereto.

In some embodiments, the antioxidant may be included in an amount of about 0.05 to about 10% by weight with respect to the total 100% by weight of the pharmaceutical preparation.

In some embodiments, a content of the antioxidant may be about 0.1 to about 5% by weight with respect to the total 100% by weight of the pharmaceutical preparation, for example, particularly about 0.1% by weight, about 0.2% by weight, about 0.3% by weight, about 0.4% by weight, about 0.5% by weight, about 1% by weight, about 1.5% by weight, about 2% by weight, about 2.5% by weight, about 3% by weight, about 3.5% by weight, about 4% by weight, about 4.5% by weight, or about 5% by weight with respect to the total 100% by weight of the pharmaceutical preparation.

In one example, the pharmaceutical preparation according to the present invention may include at least one selected from the group consisting of (a) a skin permeation enhancer, (b) a preservative, and (c) an antioxidant as a pharmaceutically acceptable excipient different from the oleaginous base, along with (S)-N-(4-(1-(2-cyanoacetyl)-3-methyl-1,2,3,6-tetrahydropyridin-4-yl)-1H-pyrrolo[2,3-b]pyridin-6-yl)cyclopropanecarboxamide or the pharmaceutically acceptable salt thereof as an active ingredient; and the oleaginous base.

The pharmaceutical preparation according to the present invention may include (S)-N-(4-(1-(2-cyanoacetyl)-3-methyl-1,2,3,6-tetrahydropyridin-4-yl)-1H-pyrrolo[2,3-b]pyridin-6-yl)cyclopropanecarboxamide or the pharmaceutically acceptable salt thereof as an active ingredient. The "pharmaceutically acceptable salt" may refer to the salts conventionally used in a pharmaceutical industry, for example, inorganic ion salts prepared from sodium, magnesium, calcium, etc.; inorganic acid salts prepared from hydrochloric acid, nitric acid, phosphoric acid, bromic acid, etc.; organic acid salts prepared from acetic acid, citric acid, succinic acid, maleic acid, oxalic acid, benzoic acid, tartaric acid, fumaric acid, benzoic acid, propionic acid, lactic acid, etc.; sulfonic acid salts prepared from methanesulfonic acid, ethanesulfonic acid, benzenesulfonic acid, etc.; amino acid salts prepared from glycine, arginine, etc.; amine salts prepared from trimethylamine, triethylamine, ammonia, pyridine, etc., but is not limited to those listed salts.

In some embodiments, a content of (S)-N-(4-(1-(2-cyanoacetyl)-3-methyl-1,2,3,6-tetrahydropyridin-4-yl)-1H-pyrrolo[2,3-b]pyridin-6-yl)cyclopropanecarboxamide or the pharmaceutically acceptable salt in the pharmaceutical preparation may be about 0.01 to 10% by weight of the formulation based on a free base with respect to the total 100% by weight of the pharmaceutical preparation.

In some embodiments, a content of (S)-N-(4-(1-(2-cyanoacetyl)-3-methyl-1,2,3,6-tetrahydropyridin-4-yl)-1H-pyrrolo[2,3-b]pyridin-6-yl)cyclopropanecarboxamide or the pharmaceutically acceptable salt thereof in the topical formulation may be about 0.05 to about 7% by weight of the formulation based on a free base with respect to the total 100% by weight of the pharmaceutical preparation, for example, particularly about 0.05% by weight, about 0.1% by weight, about 0.2% by weight, about 0.3% by weight, about 0.4% by weight, about 0.5% by weight, about 1% by weight, about 1.5% by weight, about 2% by weight, about 2.5% by weight, about 3% by weight, about 3.5% by weight, about 4% by weight, about 4.5% by weight, about 5% by weight, about 5.5% by weight, about 6% by weight, about 6.5% by weight, or about 7% by weight with respect to the total 100% by weight of the pharmaceutical preparation.

In some embodiments, the pharmaceutical preparation according to the present invention may include (a) about 0.01 to about 10% by weight of (S)-N-(4-(1-(2-cyanoacetyl)-3-methyl-1,2,3,6-tetrahydropyridin-4-yl)-1H-pyrrolo[2,3-b]pyridin-6-yl)cyclopropanecarboxamide or the pharmaceutically acceptable salt thereof based on a free base and (b) about 90 to about 99.99% by weight of the oleaginous with respect to the total 100% by weight of the pharmaceutical preparation.

In some embodiments, the pharmaceutical preparation may include (a) about 0.01 to about 10% by weight of (S)-N-(4-(1-(2-cyanoacetyl)-3-methyl-1,2,3,6-tetrahydropyridin-4-yl)-1H-pyrrolo[2,3-b]pyridin-6-yl)cyclopropanecarboxamide or the pharmaceutically acceptable salt thereof based on a free base; (b) about 69 to about 99% by weight of the oleaginous base; and (c) about 0.1 to about 30% by weight of a skin permeation enhancer with respect to the total 100% by weight of the pharmaceutical preparation.

In some embodiments, the pharmaceutical preparation may include (a) about 0.01 to about 10% by weight of (S)-N-(4-(1-(2-cyanoacetyl)-3-methyl-1,2,3,6-tetrahydropyridin-4-yl)-1H-pyrrolo[2,3-b]pyridin-6-yl)cyclopropanecarboxamide or the pharmaceutically acceptable salt thereof based on a free base; (b) about 45 to about 99.99% by weight of the oleaginous base; (c) optionally about 0.1 to about 30% by weight of the skin permeation enhancer; (d) optionally about 0.01 to about 5% by weight of a preservative; and (e) optionally about 0.05 to about 10% by weight of an antioxidant with respect to the total 100% by weight of the pharmaceutical preparation. When at least one excipient selected from the skin permeation enhancer, the preservative, and the antioxidant is included together with the active ingredient and the oleaginous base, the content of the excipient may be appropriately controlled within the above range so as to be 100% by weight together with the active ingredient and the oleaginous base.

In some embodiments, the pharmaceutical preparation may include (a) about 0.05 to about 7% by weight of (S)-N-(4-(1-(2-cyanoacetyl)-3-methyl-1,2,3,6-tetrahydropyridin-4-yl)-1H-pyrrolo[2,3-b]pyridin-6-yl)cyclopropanecarboxamide or the pharmaceutically acceptable salt thereof based on a free base; (b) about 70 to about 99.99% by weight of the oleaginous base; (c) optionally about 1 to about 15% by weight of the skin permeation enhancer; (d) optionally about 0.05 to about 3% by weight of the preservative; and (e) optionally about 0.1 to about 5% by weight of the antioxidant with respect to the total 100% by weight of the pharmaceutical preparation. When at least one excipient selected from the skin permeation enhancer, the preservative, and the antioxidant is included together with the active ingredient and the oleaginous base, the content of the excipient may be appropriately controlled within the above range so as to be 100% by weight together with the active ingredient and the oleaginous base.

The pharmaceutical preparation for topical use according to the present invention may not include a solvent capable of dissolving the active ingredient. The solvent may be glycerin or propylene glycol. In other words, the pharmaceutical preparation for topical use according to the present invention may be a pharmaceutical preparation without glycerin and propylene glycol.

In one example, the pharmaceutical preparation for topical use according to the present invention may include water.

In one example, the pharmaceutical preparation for topical use according to the present invention may include the active ingredient, the oleaginous base, emulsifiers and water.

In one example, the pharmaceutical preparation for topical use according to the present invention may not include water.

The active ingredient included in the pharmaceutical preparation of the present invention may be a selective JAK1 inhibitor. Thus, the pharmaceutical preparation of the present invention may be used for treating and/or preventing inflammatory diseases and autoimmune diseases in which the JAK1 inhibitor shows efficacy.

In examples of the present invention, the autoimmune disease may refer to a disease showing a pathological response to an autoantigen, and for example, the autoimmune disease may be xeroderma, vitiligo (e.g., non-segmental vitiligo), itching, eczema (e.g., chronic hand eczema), alopecia areata, psoriasis, atopic dermatitis, skin rash, skin sensitization (e.g., contact dermatitis or allergic contact dermatitis), skin irritation, pemphigus vulgaris (PV), bellous pemphigoid (BP), hidradenitis suppurativa, scleroderma, discoid lupus erythematosus, etc. In examples of the present invention, the preparation may be applied to all children, adolescents, adults, or the elderly.

The pharmaceutical preparation of the present invention may be used for treating and/or preventing skin diseases.

Particularly, the pharmaceutical preparation of the present invention may be used for treating and/or preventing at least one skin disease of xeroderma, vitiligo, itching, eczema, alopecia areata, atopic dermatitis, psoriasis, skin sensitization, skin rash, and skin irritation.

The present invention may also provide a method for treating and/or preventing inflammatory diseases and autoimmune diseases, including applying the pharmaceutical formulation for topical use to a skin surface of a patient, in which the autoimmune disease may be substantially the same as described above in the preparation.

The present invention may also provide a method for treating and/or preventing at least one skin disease selected from xeroderma, vitiligo, itching, eczema, alopecia areata, atopic dermatitis, psoriasis, skin sensitization, skin rash, and skin irritation, including applying the pharmaceutical preparation for topical use to a skin disease site of a patient.

The present invention may provide a use of the pharmaceutical preparation for topical use for treating and/or preventing inflammatory diseases and autoimmune diseases, and the autoimmune diseases may be substantially the same as described above in the preparation.

In addition, the present invention may provide a use of the pharmaceutical preparation for topical use for treating and/or preventing at least one skin disease selected from xeroderma, vitiligo, itching, eczema, alopecia areata, atopic dermatitis, psoriasis, skin sensitization, skin rash, and skin irritation.

The present invention may provide a use of the pharmaceutical preparation for topical use for preparing a medication for treating and/or preventing inflammatory diseases and autoimmune diseases, and the autoimmune diseases may be substantially the same as described above in the preparation.

In addition, the present invention may provide a use of the pharmaceutical preparation for topical use for preparing a medication for treating and/or preventing at least one skin disease selected from xeroderma, vitiligo, itching, eczema, alopecia areata, atopic dermatitis, psoriasis, skin sensitization, skin rash, and skin irritation.

In the present specification, the contents described in the above preparation may be substantially equally applied to the treatment and/or prevention method and use, if not contradictory.

A method for preparing a pharmaceutical preparation for topical use according to the present invention may include
(step 1) mixing (S)-N-(4-(1-(2-cyanoacetyl)-3-methyl-1,2,3,6-tetrahydropyridin-4-yl)-1H-pyrrolo[2,3-b]pyridin-6-yl)cyclopropanecarboxamide or a pharmaceutically acceptable salt thereof, and an oleaginous base; and
(step 2) cooling the mixture obtained above.

Particularly, above step (1) may include
dispersing (S)-N-(4-(1-(2-cyanoacetyl)-3-methyl-1,2,3,6-tetrahydropyridin-4-yl)-1H-pyrrolo[2,3-b]pyridin-6-yl)cyclopropanecarboxamide or the pharmaceutically acceptable salt thereof as an active ingredient in the oleaginous base.

The dispersing may have the same meaning as suspending.

The dispersing may include:
(step 1a) melting the oleaginous base to obtain a melt; and
(step 1b) adding the active ingredient per se to the melt of the oleaginous base and mixing the same.

The step (1a) may include heating an oleaginous base which is a solid or semi-solid phase at room temperature to a temperature higher than room temperature. The heating may be performed at about 60 to about 85°C, for example, about 65 to about 80°C.

A non-dissolution of the active ingredient may refer to not being dissolved in a separate solvent. The non-dissolution of the active ingredient may be a solid phase and the dispersing process may be performed by adding and mixing the active ingredient per se in the melt of the oleaginous base. Subsequently, a cooling process may be performed to prepare the pharmaceutical preparation for topical use according to the present invention.

In the present invention, the pharmaceutical preparation may be prepared by dispersing the active ingredient including (S)-N-(4-(1-(2-cyanoacetyl)-3-methyl-1,2,3,6-tetrahydropyridin-4-yl)-1H-pyrrolo[2,3-b]pyridin-6-yl)cyclopropanecarboxamide or the pharmaceutically acceptable salt thereof in the oleaginous base, so as to stably load a more amount of the active ingredient.

In one example, above step (1) may be a step of mixing (S)-N-(4-(1-(2-cyanoacetyl)-3-methyl-1,2,3,6-tetrahydropyridin-4-yl)-1H-pyrrolo[2,3-b]pyridin-6-yl)cyclopropanecarboxamide or the pharmaceutically acceptable salt thereof, a oleaginous base, and a pharmaceutically acceptable excipient different from the oleaginous base.

In one example, in above step (1b), a pharmaceutically acceptable excipient different from the oleaginous base may be mixed in the oleaginous base together with the active ingredient.

In one example, above step (2) may be performed at a temperature lower than above step (1), particularly above step (1a) and step (1b).

The inventors of the present invention confirmed that when the preparation is prepared by dissolving (S)-N-(4-(1-(2-cyanoacetyl)-3-methyl-1,2,3,6-tetrahydropyridin-4-yl)-1H-pyrrolo[2,3-b]pyridin-6-yl)cyclopropanecarboxamide or the pharmaceutically acceptable salt thereof as an active ingredient in a solvent and then mixing the resulting solution with the oleaginous base, the formulation is discolored over time, and a degree of discoloration increases as a content of the active ingredient increases. In other words, a pharmaceutical preparation for topical use suitable as a drug may not be provided by a method of dissolving (S)-N-(4-(1-(2-cyanoacetyl)-3-methyl-1,2,3,6-tetrahydropyridin-4-yl)-1H-pyrrolo[2,3-b]pyridin-6-yl)cyclopropanecarboxamide or the pharmaceutically acceptable salt thereof as an active ingredient in the solvent and then mixing the resulting solution with the oleaginous base.

The present inventors have tried to solve the above problems, thereby confirming that when the preparation is provided by dispersing (suspending) (S)-N-(4-(1-(2-cyanoacetyl)-3-methyl-1,2,3,6-tetrahydropyridin-4-yl)-1H-pyrrolo[2,3-b]pyridin-6-yl)cyclopropanecarboxamide or the pharmaceutically acceptable salt thereof as an active ingredient in the oleaginous base, even a high content of the active ingredient may be loaded without a change in the appearance of the topical formulation, and stable even under accelerated conditions (40°C, 75% RH, six months).

According to the present invention, the topical formulation may be stably prepared through a simple method while containing a high content of the active ingredient, and has shown a better therapeutic effect than a placebo formulation without a main ingredient in an atopic dermatitis animal model.

It was confirmed that the pharmaceutical preparation for topical use according to the present invention has excellent content uniformity of the drug despite being prepared without a separate solvent.

In the present specification, the contents described in the preparation may be applied to the method of preparing the preparation if not contradictory.
1) The present invention may provide the pharmaceutical preparation for topical use including (S)-N-(4-(1-(2-cyanoacetyl)-3-methyl-1,2,3,6-tetrahydropyridin-4-yl)-1H-pyrrolo[2,3-b]pyridin-6-yl)cyclopropanecarboxamide or a pharmaceutically acceptable salt thereof and an oleaginous base.
2) In 1), the pharmaceutical preparation may be ointment, cream, lotion, foam or suspension.
3) In 1) or 2), the pharmaceutical preparation may be the ointment.
4) In any one of 1) to 3), the oleaginous base may be at least one selected from a semi-solid hydrocarbon mixture, oil, wax, and fatty acid.
5) In 4), said semi-solid hydrocarbon mixture may be at least one selected from white petrolatum and purified lanolin.
6) In 4) or 5), the oil may be at least one selected from liquid paraffin, squalane, medium chain triglyceride, olive oil, castor oil, soybean oil, and liquid lanolin.
7) In any one of 4) to 6), the wax may be at least one selected from white wax, paraffin wax, and carnauba wax.
8) In any one of 4) to 7), the fatty acid may be at least one selected from cocoa butter, shea butter, and lanolin acid.
9) In any one of 1) to 8), the pharmaceutical preparation may further include a pharmaceutically acceptable excipient different from the oleaginous base, in which the excipient may be at least one selected from skin permeation enhancers, antioxidants, preservatives, chelating agents, emulsifiers, pH adjusters, stabilizers, fragrances, and colorants.
10) In any one of 1) to 9), above (S)-N-(4-(1-(2-cyanoacetyl)-3-methyl-1,2,3,6-tetrahydropyridin-4-yl)-1H-pyrrolo[2,3-b]pyridin-6-yl)cyclopropanecarboxamide or the pharmaceutically acceptable salt thereof may be included in an amount of 0.01 to 10% by weight based on a free base with respect to the total 100% by weight of the pharmaceutical preparation.
11) In any one of 1) to 10), the oleaginous base may be included in an amount of 45 to 99.99% by weight with respect to the total 100% by weight of the pharmaceutical preparation.
12) In any one of 1) to 11), the pharmaceutical preparation may further include 0.1 to 30% by weight of the skin permeation enhancer with respect to the total 100% by weight of the pharmaceutical preparation.
13) In any one of 1) to 12), the pharmaceutical preparation may further include 0.01 to 5% by weight of the preservative with respect to the total 100% by weight of the pharmaceutical preparation.
14) In any one of 1) to 13), the pharmaceutical preparation may further include 0.05 to 10% by weight of the antioxidant with respect to the total 100% by weight of the pharmaceutical preparation.
15) In any one of 1) to 14), the pharmaceutical preparation may be for treating or preventing inflammatory and autoimmune diseases, and for example, the autoimmune disease may be xeroderma, vitiligo (e.g., non-segmental vitiligo), itching, eczema (e.g., chronic hand eczema), alopecia areata, psoriasis, atopic dermatitis, skin rash, skin sensitization (e.g., contact dermatitis or allergic contact dermatitis), skin irritation, pemphigus vulgaris (PV), bellous pemphigoid (BP), hidradenitis suppurativa, scleroderma, discoid lupus erythematosus, etc.
16) In any one of 1) to 15), the pharmaceutical preparation may be for treating or preventing at least one skin disease selected from xeroderma, vitiligo, itching, eczema, alopecia areata, atopic dermatitis, psoriasis, skin sensitization, skin irritation, and skin rash.
17) The present invention may provide a method for preparing a pharmaceutical preparation for topical use, the method comprising:
   (step 1) mixing (S)-N-(4-(1-(2-cyanoacetyl)-3-methyl-1,2,3,6-tetrahydropyridin-4-yl)-1H-pyrrolo[2,3-b]pyridin-6-yl)cyclopropanecarboxamide or a pharmaceutically acceptable salt thereof; and an oleaginous base; and
   (step 2) cooling the mixture obtained above.
18) In 17), above step (1) may be a step of dispersing (S)-N-(4-(1-(2-cyanoacetyl)-3-methyl-1,2,3,6-tetrahydropyridin-4-yl)-1H-pyrrolo[2,3-b]pyridin-6-yl)cyclopropanecarboxamide or the pharmaceutically acceptable salt thereof as an active ingredient in the oleaginous base.
19) In 17) or 18), above step (1) may be a step of dispersing (S)-N-(4-(1-(2-cyanoacetyl)-3-methyl-1,2,3,6-tetrahydropyridin-4-yl)-1H-pyrrolo[2,3-b]pyridin-6-yl)cyclopropanecarboxamide or the pharmaceutically acceptable salt thereof as an active ingredient in a melt of the oleaginous base.
20) In any one of 17) to 19), above step (1) may be a step of dispersing a pharmaceutically acceptable excipient different from the oleaginous base in the melt of the oleaginous base.
21) In any one of 17) to 20), the pharmaceutical preparation may be a preparation according to any one of 1) to 16).
22) The present invention may provide a method for treating or preventing inflammatory and autoimmune diseases, the method including applying the pharmaceutical preparation according to any one of 1) to 16) to a subject in need thereof, in which the autoimmune disease may refer to a disease showing a pathological response to an autoantigen, and for example, the autoimmune disease may be xeroderma, vitiligo (e.g., non-segmental vitiligo), itching, eczema (e.g., chronic hand eczema), alopecia areata, psoriasis, atopic dermatitis, skin rash, skin sensitization (e.g., contact dermatitis or allergic contact dermatitis), skin irritation, pemphigus vulgaris (PV), bellous pemphigoid (BP), hidradenitis suppurativa, scleroderma, discoid lupus erythematosus, etc.
23) The present invention may provide a method for treating or preventing at least one skin disease selected from xeroderma, vitiligo, itching, eczema, alopecia areata, atopic dermatitis, psoriasis, skin sensitization, skin irritation, and skin rash, the method including applying the pharmaceutical preparation according to any one of 1) to 16) to a subject in need thereof.
24) The present invention may provide a use of the pharmaceutical preparation for topical use according to any one of 1) to 16) for treating or preventing inflammatory and autoimmune diseases, in which the autoimmune disease may refer to a disease showing a pathological response to an autoantigen, and for example, the autoimmune disease may be xeroderma, vitiligo (e.g., non-segmental vitiligo), itching, eczema (e.g., chronic hand eczema), alopecia areata, psoriasis, atopic dermatitis, skin rash, skin sensitization (e.g., contact dermatitis or allergic contact dermatitis), skin irritation, pemphigus vulgaris (PV), bellous pemphigoid (BP), hidradenitis suppurativa, scleroderma, discoid lupus erythematosus, etc.
25) The present invention may provide a use of the pharmaceutical preparation for topical use according to any one of 1) to 16) for treating or preventing at least one skin disease selected from xeroderma, vitiligo, itching, eczema, alopecia areata, atopic dermatitis, psoriasis, skin sensitization, skin irritation, and skin rash.
26) The present invention may provide a use of the pharmaceutical preparation for topical use according to any one of 1) to 16) for preparing a medication for treating or preventing inflammatory diseases and autoimmune diseases, in which the autoimmune disease may refer to a disease showing a pathological response to an autoantigen, and for example, the autoimmune disease may be xeroderma, vitiligo (e.g., non-segmental vitiligo), itching, eczema (e.g., chronic hand eczema), alopecia areata, psoriasis, atopic dermatitis, skin rash, skin sensitization (e.g., contact dermatitis or allergic contact dermatitis), skin irritation, pemphigus vulgaris (PV), bellous pemphigoid (BP), hidradenitis suppurativa, scleroderma, discoid lupus erythematosus, etc.
27) The present invention may provide a use of the pharmaceutical preparation for topical use according to any one of 1) to 16) for preparing a medication for treating or preventing at least one skin disease selected from xeroderma, vitiligo, itching, eczema, alopecia areata, atopic dermatitis, psoriasis, skin sensitization, skin irritation, and skin rash.
28) In any one of 1) to 27), the pharmaceutical preparation may not include a solvent.
29) In any one of 1) to 28), the pharmaceutical preparation may not include a solvent, in which the solvent may be glycerin, propylene glycol, or a mixture thereof.
30) In any one of 1) to 29), the pharmaceutical preparation may be a preparation which is substantially free or free of glycerin, propylene glycol or a mixture thereof.
31) In any one of 1) to 30), the pharmaceutical preparation may include (S)-N-(4-(1-(2-cyanoacetyl)-3-methyl-1,2,3,6-tetrahydropyridin-4-yl)-1H-pyrrolo[2,3-b]pyridin-6-yl)cyclopropanecarboxamide or a pharmaceutically acceptable salt thereof as an active ingredient; and
   at least one oleaginous base selected from the group consisting of white petrolatum, liquid paraffin, and white wax.
32) In any one of 1) to 31), a pharmaceutically acceptable salt of (S)-N-(4-(1-(2-cyanoacetyl)-3-methyl-1,2,3,6-tetrahydropyridin-4-yl)-1H-pyrrolo[2,3-b]pyridin-6-yl)cyclopropanecarboxamide may be a phosphate of (S)-N-(4-(1-(2-cyanoacetyl)-3-methyl-1,2,3,6-tetrahydropyridin-4-yl)-1H-pyrrolo[2,3-b]pyridin-6-yl)cyclopropanecarboxamide.

### Advantageous Effects

In a pharmaceutical preparation for topical use, a use thereof, and a method for preparing the same according to the present invention, the pharmaceutical preparation for topical use of the present invention has an excellent therapeutic effect in an atopic dermatitis animal model, has excellent skin permeability, and has little or no side effects (excellent safety). The pharmaceutical preparation for topical use according to the present invention can be stably prepared while containing a high content of the active ingredient by a simple method.

The pharmaceutical preparation for topical use according to the present invention can secure an excellent content uniformity of a drug even when prepared without a separate solvent, exhibit excellent stability due to no discoloration and little or no generation of impurities during storage, and maintain excellent stability regardless of a storage container.

### Brief Description of the Drawings

FIG. 1 is a view showing photographs confirming a change in the appearances of various topical formulations prepared according to Preparation Example 1 over time.
FIG. 2 is a view showing photographs of lesions of an atopic dermatitis animal model, which serves as a comparison between lesions of mice to which placebo, suspension, and a topical formulation of Example 1 were applied twice a day for 30 days and lesions of mice to which the topical formulation was not applied (normal or DNCB induction).
FIG. 3 is a view showing atopic dermatitis scores over time for each group in an atopic dermatitis animal model.
FIG. 4 is a view showing skin thickness over time for each group in an atopic dermatitis animal model.
FIG. 5 is a view showing a permeation amount of main ingredients over time when the topical formulations of the present invention and the comparative formulations are applied to a Franz diffusion cell in which the skin of a hairless mouse is inserted.
FIG. 6 is a view showing atopic dermatitis scores over time for each group in an atopic dermatitis animal model.
FIG. 7 is a view showing skin thickness over time for each group in an atopic dermatitis animal model.

### Mode for Invention

Hereinafter, the present invention will be described in more detail through exemplary embodiments. However, these exemplary embodiments are provided only for the purpose of illustrating the present invention, and thus the scope of the present invention is not limited thereto.

### Preparation Example 1. Preparing of various topical formulations

Topical formulations according to Comparative Examples and Examples were prepared as various forms of topical formulations having (S)-N-(4-(1-(2-cyanoacetyl)-3-methyl-1,2,3,6-tetrahydropyridin-4-yl)-1H-pyrrolo[2,3-b]pyridin-6-yl)cyclopropanecarboxamide phosphate as an active ingredient by using various pharmaceutically acceptable excipients according to Table 1 below.

**[Table 1]**

| | **Comparative Example 1** | **Comparative Example 2** | **Example 1** | **Example 2** | **Example 3** | **Example 4** |
|---|---|---|---|---|---|---|
| **Raw material** | Batch weight (% w/w) | | | | | |
| **Active ingredient** | 0.5 | 0.1 | 1.9 | 1.9 | 1.9 | 1.9 |
| **White petrolatum** | 71.35 | 71.75 | 88.1 | 93.1 | 85.1 | 40 |
| **Liquid paraffin** | 18.15 | 18.15 | 10 | - | - | 5 |
| **White wax** | - | - | - | 5 | 8 | 5 |
| **Glycerin** | 10 | | - | - | - | - |
| **Propylene Glycol** | - | 10 | - | - | - | - |
| **Stearyl alcohol** | - | - | - | - | 2.5 | - |
| **Span83** | - | - | - | - | 2.5 | - |
| **Glyceryl mono Stearate** | - | - | - | - | - | 3 |
| **Stearic acid** | - | - | - | - | - | 7 |
| **Cetyl alcohol** | - | - | - | - | - | 10 |
| **Purified water** | - | - | - | - | - | 28.1 |
| **Total** | 100 | 100 | 100 | 100 | 100 | 100 |

| | | | | | | |
|---|---|---|---|---|---|---|
| * Active ingredient: (S)-N-(4-(1-(2-cyanoacetyl)-3-methyl-1,2,3,6-tetrahydropyridin-4-yl)-1H-pyrrolo[2,3-b]pyridin-6-yl)cyclopropanecarboxamide phosphate | | | | | | |

The topical formulations according to Comparative Examples 1 and 2, which are ointment formulations prepared by dissolving the active ingredient, were prepared by dissolving the active ingredient in glycerin or propylene glycol, which is a solvent, for each concentration of the active ingredient, mixing with white petrolatum and/or liquid paraffin melted at high temperature (68-78°C), and then cooling at room temperature.

Example 4, which is an oil-in-water type cream, was prepared by dispersing the active ingredient in an oleaginous base melted at high temperature (68-78°C) as in Examples 1 to 3 with a homogenizer, adding purified water while dispersing with the homogenizer, and cooling the resulting mixture at room temperature, and it was confirmed that discoloration does not occur even after three months of storage at room temperature.

Examples 1 to 3, which are ointments prepared without dissolving the active ingredient to increase the content of the active ingredient, were prepared by dispersing the active ingredient in an oleaginous base melted at high temperature (68-78°C) with a homogenizer and then cooling the resulting mixture at room temperature.

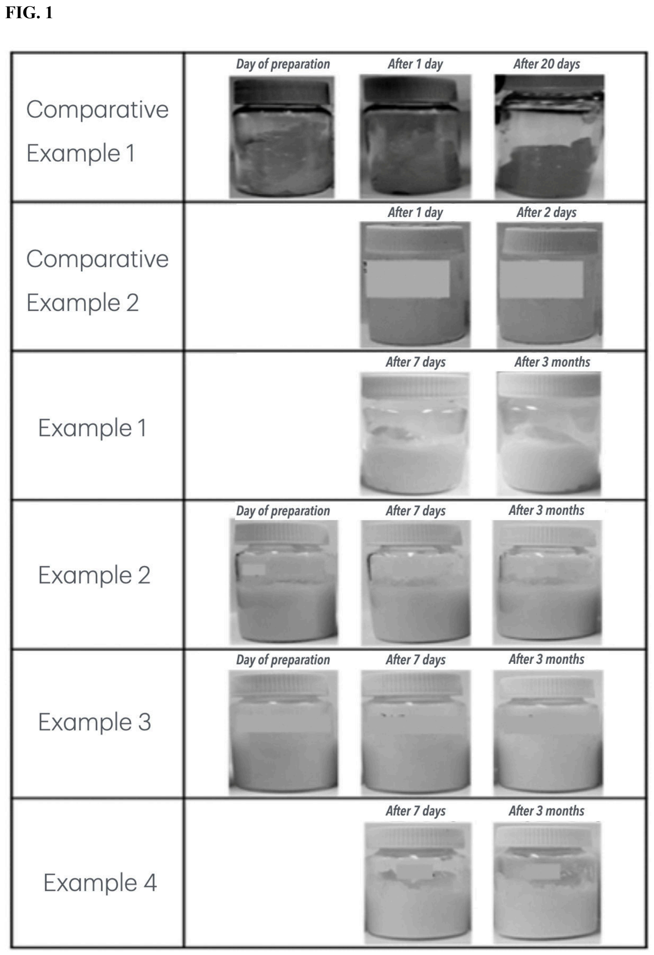

The appearances of the ointments obtained according to Examples 1 to 3 and Comparative Examples 1 and 2 were confirmed on a day of production and at a time point when predetermined days passed after the production, and the results are shown in FIG. 1.

Referring to FIG. 1, it was confirmed that the ointments obtained according to Comparative Examples 1 and 2 are discolored to become more yellow one day after storage at room temperature after the production than the formulation immediately after the production. In other words, it was confirmed that the pharmaceutical preparation for topical use suitable as a drug may not be prepared by the method of dissolving (S)-N-(4-(1-(2-cyanoacetyl)-3-methyl-1,2,3,6-tetrahydropyridin-4-yl)-1H-pyrrolo[2,3-b]pyridin-6-yl)cyclopropanecarboxamide phosphate as an active ingredient in a solvent.

On the contrary, it was confirmed that the ointments prepared according to Examples 1 to 3 has the content of the active ingredient at least three times higher than that of Comparative Examples 1 and 2, but all thereof are not discolored and maintain their appearances even after three months of storage at room temperature.

### Evaluation 1. Test for confirming efficacy in atopic dermatitis animal model

To evaluate an efficacy of the topical formulation including (S)-N-(4-(1-(2-cyanoacetyl)-3-methyl-1,2,3,6-tetrahydropyridin-4-yl)-1H-pyrrolo[2,3-b]pyridin-6-yl)cyclopropanecarboxamide phosphate, the efficacy was confirmed in the atopic dermatitis animal model.

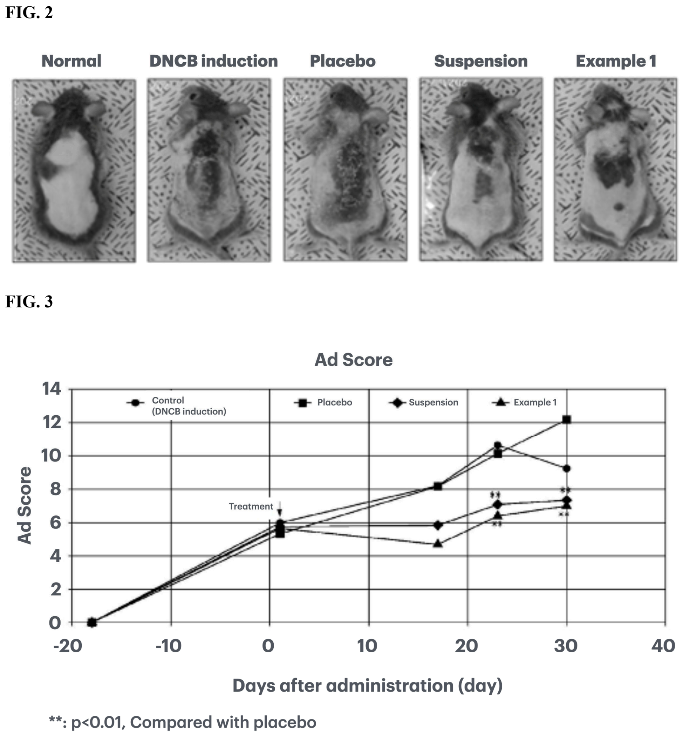

The atopic dermatitis animal model was prepared by applying dinitrochlorobenzene (DNCB) to NC/Nga mice. Test groups were divided into four groups: a control group (untreated group in which nothing was applied to a mouse having dermatitis induced with DNCB), a placebo group prepared in the same manner as in Example 1 except for the active ingredient in Example 1, a suspension group in which the active ingredient was suspended in a 0.5% methyl cellulose solution, and a group in which the ointment obtained according to Example 1 was applied, and the remaining groups except for the control group were applied twice a day for 30 days to the skin showing atopic dermatitis lesions, and then a degree of amelioration in the lesions was confirmed. The degree of amelioration in the lesions was confirmed through atopic dermatitis (AD) scores and skin thickness measurement. AD scores were determined by rating each of the five symptoms (erythema, dryness, skin edema and hematoma, erosion, and keratinization) of lesions as none (0), mild (1), moderate (2), and severe (3) and adding all scores for each of the five symptoms, and skin thickness was measured by using a caliper. The results thereof are shown in FIGS. 2 to 4.

Referring to FIGS. 2 and 3, it was confirmed that the suspension containing the active ingredient and the ointment according to Example 1 significantly improve AD scores compared to the control group and the placebo group which do not contain the main ingredient.

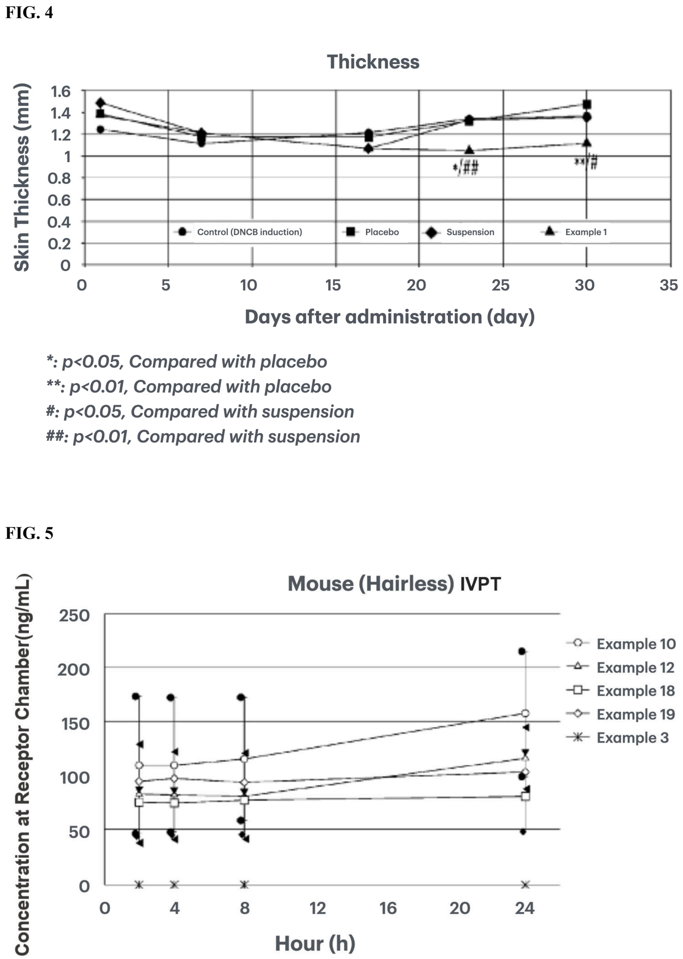

Referring to FIG. 4, in terms of skin thickness, it was confirmed that the ointment according to Example 1 significantly reduces the skin thickness when compared with the control or placebo and suspension.

### Evaluation 2. Test on stability of topical formulation

A stability test on the formulation of the ointment obtained according to Example 1 of Preparation Example 1 was carried out under two conditions of room temperature/high humidity (25°C, 60% RH) and high temperature/high humidity (40°C, 75% RH), and the results thereof are shown in Table 2 below.

**[Table 2]**

| Condition | Before test | | 1 month | | 3 months | | 6 months | |
|---|---|---|---|---|---|---|---|---|
| | Individual impurity (%) | Total impurities (%) | Individual impurity (%) | Total impurities (%) | Individual impurity (%) | Total impurities (%) | Individual impurity (%) | Total impurities (%) |
| 25°C, 60% RH | 0.03 | 0.23 | 0.03 | 0.29 | 0.04 | 0.29 | 0.04 | 0.27 |
| 40°C, 75% RH | 0.03 | 0.23 | 0.04 | 0.32 | 0.04 | 0.32 | 0.06 | 0.31 |

Referring to Table 2, it was confirmed that the ointment obtained according to Example 1 is stable even after six months under both conditions, and appearances are also maintained the same as that of an initial stage.

### Preparation Example 2: Preparation of topical formulations according to Examples 5 to 20

In order to evaluate the compositions of the excipients in the topical formulations and the properties of the formulations according to the process, tests were designed as shown in Table 3 below, and Examples 5 to 20 were prepared. (S)-N-(4-(1-(2-cyanoacetyl)-3-methyl-1,2,3,6-tetrahydropyridin-4-yl)-1H-pyrrolo[2,3-b]pyridin-6-yl)cyclopropanecarboxamide phosphate as an active ingredient was fixed to 1.9% by weight with respect to the total 100% by weight of the unit pharmaceutical preparation, and the contents of each of the liquid paraffin, the white wax, and the isopropyl myristate were as shown in Table 3, and the total amount was adjusted to 100% by weight with white petrolatum. Formulations were prepared as follows. First, all the oleaginous bases were melted at high temperature (68-78°C), and then the main ingredient was added under the operation of a homogenizer so as to be dispersed at various rates and times. After dispersion, the resulting mixture was stirred at 30°C and cooled.

**[Table 3]**

| **Example Classification** | **Name and Content of ingredients other than active ingredient (% by weight)** | | | **Preparation condition** | |
|---|---|---|---|---|---|
| | **Liquid paraffin (% by weight)** | **White wax (% by weight)** | **Isopropyl myristate (% by weight)** | **Dispersion rate (rpm)** | **Dispersion time (minute)** |
| **5** | 15 | 10 | 0 | 8000 | 5 |
| **6** | 10 | 0 | 0 | 4000 | 5 |
| **7** | 5 | 10 | 5 | 4000 | 5 |
| **8** | 10 | 5 | 5 | 6000 | 10 |
| **9** | 15 | 10 | 0 | 4000 | 10 |
| **10** | 10 | 10 | 10 | 8000 | 15 |
| **11** | 5 | 0 | 10 | 4000 | 15 |
| **12** | 15 | 5 | 10 | 8000 | 5 |
| **13** | 5 | 10 | 10 | 6000 | 5 |
| **14** | 10 | 5 | 5 | 6000 | 10 |
| **15** | 15 | 10 | 10 | 4000 | 15 |
| **16** | 15 | 0 | 0 | 6000 | 15 |
| **17** | 5 | 0 | 0 | 8000 | 5 |
| **18** | 5 | 5 | 0 | 4000 | 15 |
| **19** | 5 | 10 | 0 | 8000 | 15 |
| **20** | 15 | 0 | 10 | 4000 | 5 |

### Evaluation 3. Evaluation of composition of topical formulation and preparation and properties thereof for each process

The properties of the formulations prepared according to Examples 5 to 22 of above Table 3 are shown in Table 4 below. Viscosity, which is a value measured immediately after production (measured using a Brookfiled viscometer with No. 4 spindle), was measured and analyzed for total impurities after being left under each condition of high temperature/high humidity for a certain period of time.

**[Table 4]**

| **Example classification** | **Total impurities % (Initial)** | **Total impurities % (40°C/75%RH, two weeks)** | **Total impurities % (40°C/75%RH, four weeks)** | **Total impurities % (60°C/80%RH, two weeks)** | **Viscosity (cp)** |
|---|---|---|---|---|---|
| **5** | 0.33 | 0.28 | 0.30 | 0.51 | 411,500 |
| **6** | 0.28 | 0.30 | 0.34 | 0.58 | 188,500 |
| **7** | 0.40 | 0.30 | 0.29 | 0.68 | 472,300 |
| **8** | 0.35 | 0.30 | 0.26 | 0.53 | 262,400 |
| **9** | 0.34 | 0.32 | 0.27 | 0.33 | 566,400 |
| **10** | 0.45 | 0.32 | 0.26 | 0.45 | 364,200 |
| **11** | 0.34 | 0.31 | 0.29 | 0.75 | 162,200 |
| **12** | 0.42 | 0.30 | 0.30 | 0.64 | 217,000 |
| **13** | 0.43 | 0.30 | 0.29 | 0.67 | 335,400 |
| **14** | 0.40 | 0.29 | 0.31 | 0.56 | 259,800 |
| **15** | 0.37 | 0.29 | 0.31 | 0.69 | 330,200 |
| **16** | 0.28 | 0.34 | 0.29 | 0.60 | 197,400 |
| **17** | 0.32 | 0.34 | 0.31 | 0.39 | 223,700 |
| **18** | 0.29 | 0.28 | 0.28 | 0.59 | 395,500 |
| **19** | 0.32 | 0.29 | 0.3 | 0.55 | 505,600 |
| **20** | 0.34 | 0.32 | 0.32 | 0.42 | 105,300 |

As a result, the appearances of formulations according to above Examples 5 to 20 all showed a light yellow semi-solid preparation with a tendency of increasing viscosity upon increasing the content of white wax, and a tendency of decreasing viscosity upon the addition of isopropyl myristate.

The stability of the formulation was stable at all compositions.

The impurities of all the formulations were similar to those before testing at 40°C, 75% RH (a difference from initial was within the range of measurement error), and were slightly higher in numerical value even at 60°C, 80% RH, but without a significant increase.

From the results of the above examples, it can be confirmed that the dispersion time and the dispersion rate using the homogenizer do not significantly affect the characteristics of formulations.

### Evaluation 4. Container screening of topical formulations

The stability in various containers was evaluated using the topical formulation of Example 8, and the results thereof are shown in Table 5 below. The stability in each container was evaluated by purity testing after two weeks of storage of the formulations under two conditions of 40°C, 75% RH and 60°C, 80% RH.

**[Table 5]**

| Container | Before test | | 40°C, 75% RH | | 60°C, 80% RH | |
|---|---|---|---|---|---|---|
| | Individual impurity (%) | Total impurities (%) | Individual impurity (%) | Total impurities (%) | Individual impurity (%) | Total impurities (%) |
| Glass vial | 0.04 | 0.35 | 0.04 | 0.30 | 0.07 | 0.53 |
| Aluminum tube | 0.05 | 0.29 | 0.03 | 0.30 | 0.03 | 0.32 |
| PE tube | 0.05 | 0.31 | 0.03 | 0.28 | - | - |
| HDPE bottle | 0.03 | 0.30 | 0.03 | 0.29 | 0.04 | 0.34 |

Referring to Table 5, as a result of the test for the topical formulation of Example 8, it was observed that the appearances do not change at 40°C and 75% RH, and the formulations are melted at 60°C and 80% RH since a melting point of the excipients is low due to the characteristics of the excipients used, and then solidified again when taken out at room temperature. For the topical formulation of Example 8, the impurities were similar to those before the test at 40°C, 75% RH, and were slightly higher in numerical value at 60°C, 80% RH, but without a significant increase.

### Evaluation 5. Evaluation of skin permeability for each composition of topical formulation

Skin permeability was evaluated for each composition of topical formulation in a Franz-type diffusion cell using hairless mouse skin. An aqueous solution of Franz diffusion cell was 6% polyethylene glycol 20 oleyl ether solution, and a concentration of the drug was analyzed by LC-MS/MS after samples were stirred at 32°C and taken over time.

For experimental groups, Examples 10, 12, 18, and 19 were selected according to the content of white wax and the presence or absence of isopropyl myristate, and Corectim ointment (delgocitinib 0.5%) was used as Comparative Example 3. The test results thereof are shown in FIG. 5.

Referring to FIG. 5, a skin permeation amount of the active ingredient was high in Example 10 and Example 19, in which the content of white wax was high, and a permeation amount of the active ingredient in Example 10 and Example 12 including isopropyl myristate tended to increase over time. (In Comparative Example 3, which includes an active ingredient different from those of Examples, all were measured at 0 ng/mL at each time point of measurement.)

### Preparation Example 3. Preparation of topical formulations according to Examples 21 to 29

To evaluate the properties of the topical formulation according to a content of (S)-N-(4-(1-(2-cyanoacetyl)-3-methyl-1,2,3,6-tetrahydropyridin-4-yl)-1H-pyrrolo[2,3-b]pyridin-6-yl)cyclopropanecarboxamide phosphate as the active ingredient, the test was designed as in Table 6 below to prepare Examples 21 to 29. Topical formulations were prepared as follows. First, all the oleaginous bases were melted at high temperature (68-78°C), and then the main ingredient was added under the operation of the homogenizer so as to be dispersed (4,000 rpm, 15 minutes). After dispersion, the resulting mixture was stirred at 30°C and cooled.

**[Table 6]**

| **Example Classification** | **Active ingredient (% by weight)** | **Liquid paraffin (% by weight)** | **White wax (% by weight)** | **Isopropyl myristate (% by weight)** | **White petrolatum (% by weight)** |
|---|---|---|---|---|---|
| **21** | 0.12 | 5 | 8 | 10 | 76.88 |
| **22** | 5 | 5 | 1 | 10 | 79 |
| **23** | 0.3 | 5 | 15 | 10 | 69.7 |
| **24** | 0.3 | 5 | 1 | 10 | 83.7 |
| **25** | 5 | 5 | 15 | 10 | 65 |
| **26** | 2.65 | 5 | 8 | 10 | 74.35 |
| **27** | 2.65 | 5 | 0.45 | 10 | 81.9 |
| **28** | 2.65 | 5 | 15.55 | 10 | 66.8 |
| **29** | 5.18 | 5 | 8 | 10 | 71.82 |

### Evaluation 6. Preparation of topical formulation for each content of main ingredient and properties evaluation

The properties of Examples in above Table 6 are shown in Table 7 below. Viscosity, which is a value measured immediately after production (measured using a Brookfiled viscometer with No. 4 spindle), was measured and analyzed for total impurities after being left under each condition of high temperature/high humidity for a certain period of time.

**[Table 7]**

| **Example Classificatio n** | **Total impurities (Initial)** | **Total impurities % (40°C/75%RH, two weeks)** | **Total impurities % (40°C/75%RH, four weeks)** | **Total impurities % (60°C/80%RH, two weeks)** | **Viscosity (cp)** |
|---|---|---|---|---|---|
| **21** | 0.12 | 0.28 | 0.32 | 0.56 | 315,300 |
| **22** | 0.22 | 0.30 | 0.28 | 0.40 | 192,733 |
| **23** | 0.30 | 0.30 | 0.32 | 0.56 | 484,067 |
| **24** | 0.29 | 0.31 | 0.30 | 0.48 | 159,033 |
| **25** | 0.25 | 0.30 | 0.31 | 0.42 | 748,267 |
| **26** | 0.23 | 0.30 | 0.26 | 0.43 | 420,900 |
| **27** | 0.29 | 0.33 | 0.31 | 0.67 | 183,475 |
| **28** | 0.23 | 0.30 | 0.27 | 0.72 | 408,333 |
| **29** | 0.24 | 0.30 | 0.32 | 0.41 | 446,100 |

As a result, the viscosity of the formulations according to above Examples 21 to 29 tended to increase as the concentration of the main ingredient and the white wax increased. The stability of the formulations was generally stable at all compositions. During a stability test period, the appearances were no change at 40°C and 75% RH. For all the formulations, the impurities were similar to those before the test at 40°C, 75% RH, and were slightly higher in numerical value at 60°C, 80% RH, but without a significant increase.

### Preparation Example 4. Preparation of topical formulations with various main ingredient contents showing similar viscosities

In order to prepare topical formulations having different contents of the main ingredient, but having similar viscosities, the ratios of the main ingredient and white wax were set based on the test results of Preparation Examples 2 and 3, so as to prepare topical formulations according to Examples 30 to 34. The topical formulations were prepared by substantially the same preparation method as described in Preparation Example 3. The detailed composition and viscosity of the topical formulations for each main ingredient content are shown in Table 8, and it was confirmed that the contents of the main ingredient are different, but the viscosities are similar.

**[Table 8]**

| **Example classification** | **Active ingredient (% by weight)** | **Liquid paraffin (% by weight)** | **White wax (% by weight)** | **White petrolatum (% by weight)** | **Viscosity (cp)** |
|---|---|---|---|---|---|
| **30** | 0.38 | 5 | 6.62 | 88 | 441,400 |
| **31** | 0.64 | 5 | 6.37 | 88 | 477,667 |
| **32** | 1.27 | 5 | 5.73 | 88 | 476,167 |
| **33** | 3.81 | 5 | 3.19 | 88 | 470,200 |
| **34** | 6.35 | 5 | 0.65 | 88 | 444,567 |

### Evaluation 7. Test for confirming efficacy in atopic dermatitis animal model

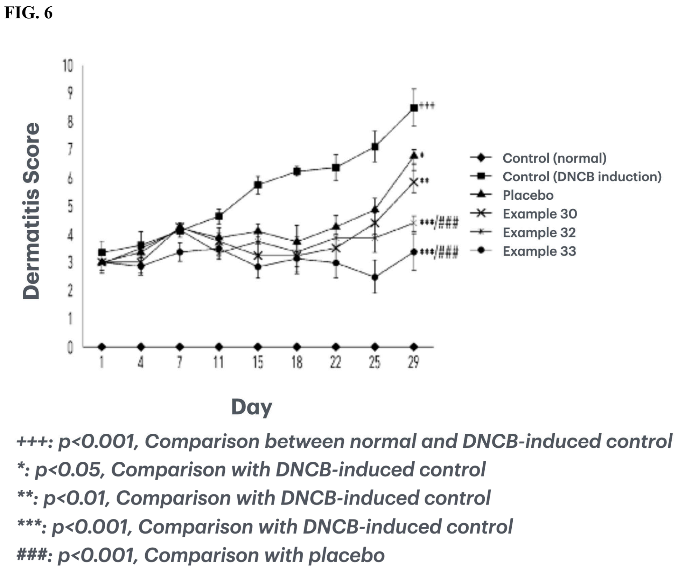

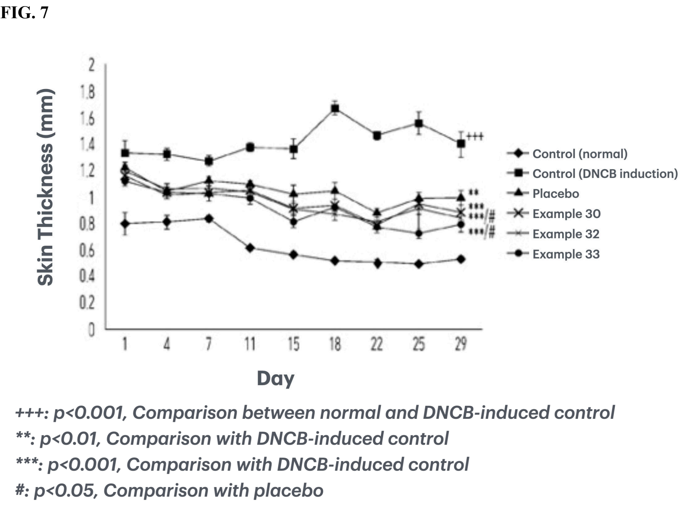

In order to evaluate the efficacy of the topical formulation according to the content of the active ingredient, the efficacy in an atopic dermatitis animal model was confirmed. The atopic dermatitis animal model was prepared by applying dinitrochlorobenzene (DNCB) to NC/Nga mice. The test groups were divided into six groups: a control group (normal), a control group (DNCB induction), a placebo group prepared as in Example 30 except for the active ingredient, a group administered with Example 30, a group administered with Example 32, and a group administered with Example 33, and the remaining groups except for the two control groups were applied to skin showing atopic dermatitis lesions twice daily for 28 days, so as to check a degree of amelioration in the lesions. The degree of amelioration in the lesions was confirmed through atopic dermatitis (AD) scores and measurement of skin thickness, and was measured by the same experimental method as described in Evaluation 1. The results thereof are shown in FIGS. 6 and 7.

The AD score results of FIG. 6 showed that the groups administered with Examples 30, 32 and 33, which are the topical formulations containing (S)-N-(4-(1-(2-cyanoacetyl)-3-methyl-1,2,3,6-tetrahydropyridin-4-yl)-1H-pyrrolo[2,3-b]pyridin-6-yl)cyclopropanecarboxamide phosphate exhibit an effect of amelioration by 13%, 35%, and 50%, respectively, compared with the placebo-administered group.

The skin thickness results of FIG. 7 showed that the groups administered with Examples 30, 32 and 33 exhibit an effect of reduction in skin thickness by 11%, 15%, and 20%, respectively, compared with the placebo-administered group.

The present invention has been described with reference to preferred exemplary embodiments herein, but it will be understood by those skilled in the art that the present invention may be variously changed and modified without departing from the spirit and field of the present invention, as described in the following scope of patent claims.

## Claims

1. A pharmaceutical preparation for topical use comprising (S)-N-(4-(1-(2-cyanoacetyl)-3-methyl-1,2,3,6-tetrahydropyridin-4-yl)-1H-pyrrolo[2,3-b]pyridin-6-yl)cyclopropanecarboxamide or a pharmaceutically acceptable salt thereof as an active ingredient, and an oleaginous base.

2. The pharmaceutical preparation according to claim 1, wherein a formulation of the pharmaceutical preparation is any one of ointment, cream, lotion, foam, and suspension.

3. The pharmaceutical preparation according to claim 1, wherein a formulation of the pharmaceutical preparation is ointment.

4. The pharmaceutical preparation according to claim 1, wherein the oleaginous base comprises at least one selected from a semi-solid hydrocarbon mixture, oil, wax, and fatty acid.

5. The pharmaceutical preparation according to claim 4, wherein said semi-solid hydrocarbon mixture comprises at least one selected from white petrolatum and purified lanolin.

6. The pharmaceutical preparation according to claim 4, wherein said oil comprises at least one selected from liquid paraffin, squalane, medium chain triglyceride, olive oil, castor oil, soybean oil, and liquid lanolin.

7. The pharmaceutical preparation according to claim 4, wherein said wax comprises at least one selected from white wax, paraffin wax, and carnauba wax.

8. The pharmaceutical preparation according to claim 4, wherein said fatty acid comprises at least one selected from cocoa butter, shea butter and lanolin acid.

9. The pharmaceutical preparation according to claim 1, wherein the pharmaceutical preparation further comprises a pharmaceutically acceptable excipient different from the oleaginous base,
wherein the excipient comprises at least one selected from skin permeation enhancers, antioxidants, preservatives, chelating agents, emulsifiers, pH adjusters, stabilizers, fragrances, and colorants.

10. The pharmaceutical preparation according to claim 1, wherein (S)-N-(4-(1-(2-cyanoacetyl)-3-methyl-1,2,3,6-tetrahydropyridin-4-yl)-1H-pyrrolo[2,3-b]pyridin-6-yl)cyclopropanecarboxamide or the pharmaceutically acceptable salt thereof is comprised in an amount of 0.01 to 10% by weight based on a free base with respect to total 100% by weight of the pharmaceutical preparation.

11. The pharmaceutical preparation according to claim 1, wherein the oleaginous base is comprised in an amount of 45 to 99.99% by weight with respect to total 100% by weight of the pharmaceutical preparation.

12. The pharmaceutical preparation according to claim 1, wherein the pharmaceutical preparation further comprises 0.1 to 30% by weight of a skin permeation enhancer with respect to total 100% by weight of the pharmaceutical preparation.

13. The pharmaceutical preparation according to claim 1, wherein the pharmaceutical preparation further comprises 0.01 to 5% by weight of a preservative with respect to total 100% by weight of the pharmaceutical preparation.

14. The pharmaceutical preparation according to claim 1, wherein the pharmaceutical preparation further comprises 0.05 to 10% by weight of an antioxidant with respect to total 100% by weight of the pharmaceutical preparation.

15. The pharmaceutical preparation according to claim 1, wherein the pharmaceutical preparation is for treating or preventing inflammatory and autoimmune diseases,
wherein the autoimmune disease is at least one selected from the group consisting of xeroderma, vitiligo, itching, eczema, alopecia areata, psoriasis, atopic dermatitis, skin rash, skin sensitization, skin irritation, pemphigus vulgaris (PV), bellous pemphigoid (BP), hidradenitis suppurativa, scleroderma, and discoid lupus erythematosus.

16. The pharmaceutical preparation according to claim 1, wherein the pharmaceutical preparation is for treating or preventing at least one skin disease selected from xeroderma, vitiligo, itching, eczema, alopecia areata, atopic dermatitis, psoriasis, skin sensitization, skin irritation, and skin rash.

17. A method for preparing a pharmaceutical preparation for topical use, the method comprising:
(step 1) mixing (S)-N-(4-(1-(2-cyanoacetyl)-3-methyl-1,2,3,6-tetrahydropyridin-4-yl)-1H-pyrrolo[2,3-b]pyridin-6-yl)cyclopropanecarboxamide or a pharmaceutically acceptable salt thereof; and an oleaginous base; and
(step 2) cooling the mixture obtained above.

18. The method according to claim 17, wherein above step (1) comprises
Dispersing (S)-N-(4-(1-(2-cyanoacetyl)-3-methyl-1,2,3,6-tetrahydropyridin-4-yl)-1H-pyrrolo[2,3-b]pyridin-6-yl)cyclopropanecarboxamide or the pharmaceutically acceptable salt thereof as an active ingredient in the oleaginous base.

19. The method according to claim 17, wherein above step (1) comprises
dispersing (S)-N-(4-(1-(2-cyanoacetyl)-3-methyl-1,2,3,6-tetrahydropyridin-4-yl)-1H-pyrrolo[2,3-b]pyridin-6-yl)cyclopropanecarboxamide or the pharmaceutically acceptable salt thereof as an active ingredient in a melt of the oleaginous base.

20. The method according to claim 19, wherein above step (1) comprises dispersing a pharmaceutically acceptable excipient different from the oleaginous base in the melt of the oleaginous base.

21. A method for treating or preventing inflammatory and autoimmune diseases, the method comprising applying the pharmaceutical preparation according to claim 1 to a subject in need thereof,
wherein the autoimmune disease is at least one selected from the group consisting of xeroderma, vitiligo, itching, eczema, alopecia areata, psoriasis, atopic dermatitis, skin rash, skin sensitization, skin irritation, pemphigus vulgaris (PV), bellous pemphigoid (BP), hidradenitis suppurativa, scleroderma, and discoid lupus erythematosus.

22. A method for treating or preventing at least one skin disease selected from xeroderma, vitiligo, itching, eczema, alopecia areata, atopic dermatitis, psoriasis, skin sensitization, skin irritation, and skin rash, the method comprising applying the pharmaceutical preparation according to claim 1 to a subject in need thereof.

23. A use of the pharmaceutical preparation for topical use according to claim 1 for treating or preventing inflammatory and autoimmune diseases,
wherein the autoimmune disease is at least one selected from the group consisting of xeroderma, vitiligo, itching, eczema, alopecia areata, psoriasis, atopic dermatitis, skin rash, skin sensitization, skin irritation, pemphigus vulgaris (PV), bellous pemphigoid (BP), hidradenitis suppurativa, scleroderma, and discoid lupus erythematosus.

24. A use of the pharmaceutical preparation for topical use according to claim 1 for treating or preventing at least one skin disease selected from xeroderma, vitiligo, itching, eczema, alopecia areata, atopic dermatitis, psoriasis, skin sensitization, skin irritation, and skin rash.

25. A use of the pharmaceutical preparation for topical use according to claim 1 for preparing a medication for treating or preventing inflammatory diseases and autoimmune diseases,
wherein the autoimmune disease is at least one selected from the group consisting of xeroderma, vitiligo, itching, eczema, alopecia areata, psoriasis, atopic dermatitis, skin rash, skin sensitization, skin irritation, pemphigus vulgaris (PV), bellous pemphigoid (BP), hidradenitis suppurativa, scleroderma, and discoid lupus erythematosus.

26. A use of the pharmaceutical preparation for topical use according to claim 1 for preparing a medication for treating or preventing at least one skin disease selected from xeroderma, vitiligo, itching, eczema, alopecia areata, atopic dermatitis, psoriasis, skin sensitization, skin irritation, and skin rash.
